(19) Europäisches Patentamt
European Patent Office
Office européen des brevets

(11) EP 3 106 515 B1

(12) **EUROPEAN PATENT SPECIFICATION**

(45) Date of publication and mention of the grant of the patent:
**21.11.2018 Bulletin 2018/47**

(21) Application number: **14879588.3**

(22) Date of filing: **02.07.2014**

(51) Int Cl.:
***C12N 1/20*** (2006.01) ***C12N 9/64*** (2006.01)
***A23C 19/032*** (2006.01) ***C12R 1/01*** (2006.01)

(86) International application number:
**PCT/CN2014/081446**

(87) International publication number:
**WO 2015/109766 (30.07.2015 Gazette 2015/30)**

# (54) METHOD FOR PREPARING FERMENTATION BROTH EXTRACTS HAVING CHYMOSIN ACTIVITY

VERFAHREN ZUR HERSTELLUNG EINES EXTRAKTES AUS EINER FERMENTATIONSBOUILLON WELCHER EINE CHYMOSINWIRKUNG AUFZEIGT

PROCÉDÉ DE PRÉPARATION D'EXTRAITS DE BOUILLONS DE FERMENTATION PRÉSENTANT UNE ACTIVITÉ CHYMOSINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2014 CN 201410040762**

(43) Date of publication of application:
**21.12.2016 Bulletin 2016/51**

(73) Proprietor: **Bright Dairy & Food Co., Ltd**
**Shanghai 201103 (CN)**

(72) Inventors:
- **HANG, Feng**
  **Shanghai 201103 (CN)**
- **GUO, Benheng**
  **Shanghai 201103 (CN)**
- **LIU, Zhenmin**
  **Shanghai 201103 (CN)**
- **CHEN, Wei**
  **Shanghai 201103 (CN)**
- **WANG, Qinbo**
  **Shanghai 201103 (CN)**
- **SONG, Xin**
  **Shanghai 201103 (CN)**
- **WU, Zhengjun**
  **Shanghai 201103 (CN)**
- **ZHANG, Hao**
  **Shanghai 201103 (CN)**
- **ZHU, Junwei**
  **Shanghai 201103 (CN)**

(74) Representative: **Isern Patentes y Marcas S.L.**
**Avda. Diagonal, 463 Bis, 2°**
**08036 Barcelona (ES)**

(56) References cited:
**EP-A1- 1 386 539** **EP-A1- 1 535 999**
**CN-A- 102 041 237** **CN-A- 103 211 035**
**CN-A- 103 740 618**

- **Heloiza Ferreira Alves-Prado ET AL: "Evaluation of solid and submerged fermentations for the production of cyclodextrin glycosyltransferase by Paenibacillus campinasensis H69-3 and characterization of crude enzyme", Applied Biochemistry and Biotechnology, 1 March 2006 (2006-03-01), pages 234-246, XP055376558, Totowa DOI: 10.1385/ABAB:129:1:234 Retrieved from the Internet: URL:https://rd.springer.com/content/pdf/10 .1385/ABAB:129:1:234.pdf**
- **SONG, XI ET AL.: 'Separation and Identification of A Chymosin Producing Bacterium from Soil of Yak Grazing District in Tianzhu Country of Gansu Province' FOOD SCIENCE vol. 30, no. 11, 31 December 2009, pages 158 - 162, XP008183905**
- **ZHANG, WEIBING ET AL.: 'Study on isolation, identification of Bacillus amyloliquefaciens producing chymosin and enzyme properties' SCIENCE AND TECHNOLOGY OF FOOD INDUSTRY 31 December 2012, pages 172 - 176 AND 180**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical field:

The present invention belongs to the field of biotechnology, and particularly relates to a method for preparing fermentation broth extracts having milk-clotting activity (MCA) and products and application thereof.

### Back ground:

Curding is a key step in the production of original cheese, wherein milk-clotting enzyme (MCE) plays a crucial role during the process and relates directly to the quality and yield of cheese. The rennet used in conventional cheese processing comes from the fourth stomach (abomasum) of a ruminant. It mainly comprises chymosin and pepsin. The commercialized calf rennet and the rennet thereof make up a proportion of 50-95%. The rennet-mediated curd reaction relates to two steps: (1) enzyme hydrolyze casein: chymosin (E.C. 3.4.23.4) has a high specificity to *k*-CN and mainly hydrolyzes the peptide bond of $Phe_{105}$-$Met_{106}$ in *k*-CN, and generates: *k*-casein macropeptide consisting of 106-109 amino acid residue and para-*k*-casein consisting of 1-105 amino acid residue, the chymosin may also hydrolyze $\alpha_{s1}$-CN, $\alpha_{s2}$-CN, $\beta$-CN; (2) once enough *k*-CNs were hydrolyzed, the para-*k*-caseins will aggregate to form three-dimensional network gel, $Ca^{2+}$ will bridge the aggregation of casein micelle, thereby triggers the unstability for casein micelles and forms the cheese curd.

Over 50 years ago, the demand for calf rennet which being used in cheese processing had already exceeded the yield thereof. Meanwhile, the world production of cheese has increased for about 3.5 times since 1961. However, the supply of calf rennet shows a decreasing trend. The calf rennet in the world can only supply 20-30% of the coagulant required for the world production of cheese at present. For the imbalance between supply and demand of calf rennet and factors such as high price, religion (Islam and Judaism), diet (vegetarianism) and food regulations, seeking alternatives thereof has become one of the hot points in the research in the field of dairy product. The research on alternatives for calf rennet mainly focuses on four aspects: animal, plant, gene recombination and microorganism. The MCE derived from animal and plant is mainly used for cheese of particular kinds, the source and yield thereof are limited likewise; the chymosin prepared with gene recombination technology has advantages such as a single component. However, recombinant chymosin is prohibited in some countries such as France, Germany and Netherlands.

Microorganism may produce many kinds of enzymes, wherein most of them are secreted rarely and relating to the process of cell metabolism, extracellular enzymes may digest the insoluble macromolecular substances such as cellulose, starch and protein, and transfer into the cell to serve as the nutrient substance for cell growth. Many of the extracellular proteases with microorganism origins, especially those derived from fungi and bacteria have properties similar to chymosin. Comparing to the chymosin, the milk-clotting enzymes (MCEs) origins from microorganism have advantages such as low production cost, wider range of biological diversity and simple method for gene modification. The microbial MCEs can be divided into two types: (a) pepsin-like with origins of *Aspergillus* spp. and *Rhizopus* spp.; (b) rennin-like with origins of *Mucor* spp., *Rhizomucor* spp. and *Endothia parasitica.* Currently, the MCEs derived from *Rhizomucor miehei,* Rhizomucor pusillus and *Endothia parasitica* have already been used in large-scale commercial production, comprising 33% of the world protease market. However, the research on bacterial MCEs mainly focuses on Bacillus in recent years, the proteases obtained usually have a high proteolytic activity and strong heat resistance which making them hard for inactivation, thus further leading the degradation of caseins and converting them into whey. Therefore, they have a negative effect on the yield of cheese and only parts of them are appropriate for the production of cheese.

In this sense, document EP1386539 discloses a method of producing a cheese-like dairy product adding at least 10 wt%, relative to the starting material milk, of water containing Paenibacillus sp. bacteria or water that has been treated with immobilized cells of Paenibacillus sp. bacteria into starting material milk, followed by maintaining at a prescribed temperature, separating off the curd thus formed, and performing productization with or without further maturing; a cheese-like dairy product thus produced; and a water purifier packed with a support having Paenibacillus sp. bacteria immobilized thereon and a filter medium as a packing material.

Likewise, document EP1535999 discloses a milk-coagulating enzyme of bacterial origin and a process for producing cheese by using this enzyme, namely, an enzyme that is produced by a bacterium belonging to the genus Paenibacillus and exhibits milk-coagulating activity, a process for producing the enzyme; a process for producing cheese; and a cheese-like food product.

However, although some of the MCEs derived from microorganism have been industrialization, screening of new MCEs-producing microbial strains is still an important research work in this filed.

**Description:**

Therefore, the technical problem to be solved by the present invention is to provide a method for preparing fermentation broth extracts having MCA and products and application thereof aimed at solving the technical problem of gravely insufficient source and yield for MCEs at present.

To solve the technical problem mentioned above, the first technical solution adopted by the present invention is a preparation method for fermentation broth extracts having MCA, wherein the preparation method comprises the steps of: obtaining the fermentation broth by shaking culture Paenibacillus (Paenibacillus bovis sp. nov.) CGMCC No.8333 at 15°C-40°C for 18-120 hours using a wheat bran broth, and obtaining the extracts by taking out the supernatant after centrifuging the resulting fermentation broth.

The wheat bran broth according to the present invention comprises wheat bran and water. Wherein, the wheat bran is conventional wheat bran in the field, and the preferred origins of the wheat bran comprises Shandong, Henan and Jiangsu Province, and the further preferred origin is Shandong Province.

The content of wheat bran in the wheat bran broth according to the present invention preferably ranges within 1%-14%, more preferably ranges within 1%-10%, and most preferably is 3%, wherein these percentages refer to mass percentages. The inoculum size of the strain *Paenibacillus* CGMCC No.8333 preferably ranges within 1-9%, more preferably ranges within 3%-7%, and most preferably is 5%, wherein these percentages refer to the volume percentages. The culture temperature preferably ranges within 20°C-40°C, more preferably ranges within 25°C-35°C and most preferably is 30°C. The shaking speed preferably ranges within 100-300r/min, the more preferably ranges within 140-300 r/min and especially more preferably ranges within 200-300r/min, and most preferably is 300r/min. The duration for the culture preferably ranges within 18-120 hours, more preferably ranges within 20-48 hours, and most preferably is 20 hours, wherein the speed for the centrifugation preferably ranges within 9000-14000 r/min.

The preferred nutrient contents comprised in the wheat bran broth according to the present invention are protein: 18.6%, fat: 6.20%, total carbohydrate: 63.9%, moisture: 7.89%, and ash: 3.38%, wherein the percentages refer to mass percentages.

The preparation method for the fermentation broth extracts having MCA according to the present invention also comprises a step of the activation of the strain. The step of activation of the strain comprises inoculating the *Paenibacillus* CGMCC No.8333 according to the present invention into the TYC broth for shaking culture at 25°C-35°C and 180 r/min for 18-48 hours to obtain seeds; and inoculating the activated strain into the 250mL-Erlenmeyer flask containing 1%-14% of wheat bran broth with an inoculum size of 1-9% for shaking culture, wherein the liquid volume of the 250mL-Erlenmeyer flask ranges within 20mL-100mL, the speed of the shaker ranges within 140-300r/min, the culture temperature ranges within 20°C-40°C and the duration for the culture ranges within 18-120 hours, wherein these percentages refer to the mass percentages.

Wherein, the inoculum size of the strain *Paenibacillus* CGMCC No.8333 more preferably ranges within 1-5%, and most preferably is 3%, and these percentages refer to volume percentages. The content of wheat bran in the wheat bran broth preferably ranges within 1%-14%, more preferably ranges within 1%-10% and most preferably is 3%, and these percentages refer to the mass percentages. The liquid volume of the 250mL Erlenmeyer flask preferably ranges within 20mL-100mL, more preferably ranges within 25mL-75mL and most preferably is 30mL. The shaking speed for the shaking culture preferably ranges within 140-300 r/min and most preferably is 300r/min. The temperature for the culture preferably ranges within 25°C-35°C and most preferably is 30°C. The duration for the activation preferably ranges within 18-48 hours, more preferably ranges within 18-24 hours and most preferably is 18 hours.

The preparation method for the fermentation broth extracts having MCA according to the present invention also comprises the step of refining and drying the supernatant obtained, wherein the step of refining and drying refers to a conventional vacuum freeze drying step in the field, and the temperature for the vacuum freeze drying preferably ranges within -44°C to -40°C, and more preferably is -40°C.

The TYC broth according to the present invention refers to a conventional TYC broth in the field, and the preferred recipe and preparation method for the TYC broth is: 15 g of casein peptone or tryptone, 50 g of sucrose, 5.0 g of yeast extract, 0.2 g of L-cystine, 20 g of sodium acetate, 0.1 g of $Na_2SO_4$, 1 g of NaCl, 2 g of $Na2HPO_4 \cdot 12H_2O$, 2 g of $NaHCO_3$, and water was added to the volume of 1 L while pH value was adjusted to 7.3, sterilized for 15 minutes at 121°C to obtain the culture medium. The TYC agar was prepared as the recipe of TYC broth and supplemented with 15-20g of agar. The preparation method from the raw materials mentioned above is a conventional preparation method in the field, and all raw materials are commercially available.

To solve the technical problem mentioned above, the second technical solution according to the present invention is the fermentation broth extracts having MCA obtained by the preparation method according to the present invention. The MCA of the fermentation broth extracts prepared by the present invention is higher than that of the fermentation broth extracts with ordinary microorganism origins.

To solve the technical problem mentioned above, the third technical solution according to the present invention is use of the fermentation broth extracts having MCA according to the present invention in the preparation of MCE.

The preparation method for MCE according to the present invention is a conventional preparation method in the field, which only requires the fermentation broth extracts having MCA obtained by the present invention as the raw material for preparation to obtain the MCE.

To solve the technical problem mentioned above, the fourth technical solution according to the present invention is use of the fermentation broth extracts having MCA according to the present invention in the preparation of fermented dairy products.

The fermented dairy products according to the present invention refer to conventional fermented dairy products in this field, which preferably comprise fermented dairy, lactobacillus beverages, yogurt powders, fermented cheese, and more preferably comprise fermented cheese and even more preferably comprise cheese.

In conformity with common knowledge in this field, it is possible to combine the preferred conditions mentioned above discretionarily to obtain various preferred embodiments of the present invention.

All reagents and raw materials used in the present invention are commercially available.

The positive effects and progress of the present invention lie in that the MCA of the fermentation broth extracts according to the present invention is up to 6469.72±280.65 SU/mL, and the protelytic activity of the obtained fermentation broth extracts is merely 10.54±0.65 U/mL, with the ratio between the two beings up to 613.82. The fermentation broth extracts having MCA according to the present invention may enable the liquid milk to curdle in a short time, and the fermentation broth extracts obtained has a very low non-specific hydrolytic activity for casein. Therefore the fermentation broth extracts having MCA has a broad application prospect in the production and processing of original cheese.

## Preservation Information of Biological Materials

The *Paenibacillus* spp. BD3526 of the present invention has been preserved in China General Microbiological Culture Collection Center (CGMCC) since October 14, 2013, the address for preservation is: Institute of Microbiology, Chinese Academy of Sciences, NO.1 West Beichen Road, Chaoyang District, Beijing 100101, China. The preservation number for the strain is: CGMCC No.8333. The systematic name of the strain is *Paenibacillus bovis* sp. nov., and the number of culture bank of Bright Dairy & Food Co., Ltd is BD3526.

## Breif description of the drawings:

Characteristics and beneficial effects of the present invention are illustrated below through drawings.

FIG. 1 shows the colonies of the *Paenibacillus* CGMCC No.8333 on TYC agar according to the present invention.

FIG. 2 shows the phylogenetic tree of 16S rRNA of the *Paenibacillus* CGMCC No.8333 according to the present invention.

FIG. 3 shows the form of curd clotted by the supernatant of the wheat bran broth fermented by *Paenibacillus* CGMCC No.8333 according to the present invention.

## Detailed description of preferred embodiments:

The present invention is further illustrated by means of embodiments below; however, the present invention is not limited within the scope of the embodiments consequently. For experimental methods without any indicated specific conditions, conventional methods and conditions may be preferred. Otherwise, a descriptive literature shall be used for reference upon selection. The room temperature according to the present invention refers to the temperature of operating room for the experiment and usually ranges within a scope of 25°C. *Paenibacillus hunanensis* FeL05$^T$ (ACCC 10718$^T$= CGMCC No. 1.8907 $^T$) and *Paenibacillus polymyxa* ATCC 842$^T$ (CGMCC No.1.4261$^T$) are purchased from China General Microbiological Culture Collection Center (CGMCC, the address is: Institute of Microbiology, Chinese Academy of Sciences, NO.1 West Beichen Road, Chaoyang District ,Beijing 100101, China).

Embodiment 1 the preliminary screening of strain BD3526

The yak milk is collected at Damxung County, Tibet Autonomous Region. Taking 1ml of the milk with aseptic technique for serial dilution with sterile normal saline, applying the diluent onto the solid TYC culture medium by means of smearing (the composition of the TYC culture medium comprises: 15g of casein peptone or tryptone , 50g of sucrose, 5.0g of yeast extract, 0.2g of L-cystine, 20g of sodium acetate, 0.1g of $Na_2SO_4$, 1g of NaCl, 2g of $Na_2HPO_4 \cdot 12H_2O$ and 15-20g of agar with water added to the volume of 1L while pH value adjusted to 7.3, dissolving all these mentioned above with distilled water and sterilizing for 15 minutes at 121°C to obtain the culture medium), then culturing it for 24-48 hours at 30°C. Selecting several nasal mucus-like single colonies with good string performance and transferring them respectively onto the new solid TYC culture media to obtain the purified colonies.

Embodiment 2 the obtaining of strain BD3526 and the characteristics thereof

Biolog microbial automatic detector (manufacturer: Biolog) identification test:

Biolog microbial automatic detector (manufacturer: Biolog) identification test is the original carbon source utilization method of Biolog, which utilizing the differences in metabolism for microorganisms adopting 95 kinds of carbon sources and other chemical substances. The microorganism was inoculated onto the 96-well plate and incubated it for a period (A1 well is the negative control). According to the absorbance and the turbidity caused by the growth of microorganism in the 96-well plate to generate the characteristic fingerprint, comparing with the standard strain profiles database to obtain the result of final identification.
1) Taking several single colonies according to Embodiment 1 mentioned above and inoculating them into the liquid TYC respectively, then incubating for 24h at 30°C and 180r/min.
2) Centrifuging them for 20min in the speed of 10000r/min and removing the supernatant, adding 1mL of normal saline and shaking them on a shaker for 5min for homogenized mixing; centrifuging them for 20min in the speed of 10000r/min again and repeat the processes for 2 times to remove the carbon sources therein; removing the supernatant, adding 1mL of normal saline and shaking them on a shaker for 5min for homogenized mixing, then centrifuging them for 1min in the speed of 2000r/min.
3) Pouring the supernatant into a test tube with 20mL of sterile normal saline (NaCl, 0.85%) and keeping its $OD_{590}$ at $0.13 \pm 0.02$.
4) Adding the diluent onto the Biolog ECO micro plate (150 μL / well), and incubating it at 20°C, reading the data by Biolog bacteria automatic readout device every 12h for a 2d-continuous determination. The result is shown in Table 1.

Table 1 Biolog identification result for strain BD3526

| | PROB | SIM | DIST | Varieties |
|---|---|---|---|---|
| 1 | 0.535 | 0.535 | 6.927 | *Virgibacillus sediminis* |
| 2 | 0.117 | 0.117 | 7.531 | *Brachybacterium paraconglomeratum* |
| 3 | 0.102 | 0.102 | 7.716 | *Paenibacillus tundrae* |
| 4 | 0.095 | 0.095 | 7.802 | *Paenibacillus polymyxa* |

The result of identification shall take 3 parameters into consideration: Probability (PROB), Similarity (SIM) and Distance (DIS). SIM and DIS values are 2 important parameters, indicating the matching degree between testing result and the corresponding data in database. In the case of DIS<5.0 and SIM>0.75, the result is regarded as a good matching. The result shows that the determined SIM value for strain BD3526 is 0.535<0.75, indicating a low matching degree and a large difference in metabolic characteristic to the strains within the database. It is thus likely to be a new species or genus of microorganism.
The strain BD3526 obtained has been preserved in China General Microbiological Culture Collection Center (CGMCC) since October 14, 2013, the address of which is: Institute of Microbiology, Chinese Academy of Sciences, NO.1 West Beichen Road, Chaoyang District, Beijing 100101, China. The preservation number for the strain is: CGMCC No.8333. The systematic name of the strain is *Paenibacillus bovis* sp. nov., and name of the strain is BD3526.

Embodiment 3 the characteristics of strain BD3526

1. Colony characteristics:

The single colonies of strain BD3526 onto the solid TYC culture media (agar) were transferred and incubated at 30°C for 24h, 36h and 48h respectively. The characteristics of colonies such as size, color, edge, embossment, smoothness, viscidity and transparency were observed respectively. The result is shown in FIG. 1. The result shows that the strain BD3526 forms smooth, mucous and non-transparent colonies with regular edge and a diameter of 3-5mm on TYC agar.

2. Morphological observation and physiological and biochemical characteristics:

Pick up the fresh cultures from solid TYC culture medium (agar) after a 24-hour incubation for physiological and biochemical tests. The result shows that BD3526 is a Gram-positive *bacillus* with endospore, in the shape of oval and no swelling. The parameters for physical and chemical reactions of BD3526 are shown in Table 2.

Table 2 Result for physical and chemical test on strain BD3526

| | | |
|---|---|---|
| Oxidase- | Contact enzyme+ | β-galactosidase+ |
| Double hydrolysis of arginine- | Lysine decarboxylase- | Ornithine decarboxylase+ |
| Urease- | Citrate utilization- | Nitrate reduction+ |
| Indole production- | VP reaction+ | $H_2S$ production- |
| Aamylolysis+ | Esculin hydrolysis+ | Gelatin liquefaction+ |

3. API 50 CHB identification characteristics

Determine the acid producing from fermented carbohydrate utilized by strain BD3526 by using API 50 CHB identification system (manufacturer: bioMe' rieux). For the reagent strips of API 50 CHB, different serial number corresponds to different carbon source; meanwhile, indicator is contained therein. Thus in case the corresponding carbon source is used, the pH value of culture solution will decrease and the color of the indicator will change to provide convenience for observing and recording.

1) The components comprised in API 50 CHB basic culture medium: 1g of tryptone, 0.5g of yeast extract, 2g of ammonium sulfate, 0.18g of phenol red and 10ml of inorganic salt base (Cohen-Bazire) and 1000ml of phosphate buffer (pH7.8).

2) Culturing the strain on plates and picking out several plates with separated single colonies in similar sizes. Picking up single colonies in similar sizes into the culture medium of 1) to form the bacterial suspension with $OD_{600}$=0.4-0.6.

3) Adding the inoculated API 50 CHB culture medium into the tubes containing test strips and covering them with a layer of sterile paraffin oil.

4) Incubating them at 30°C and recording the results at 24 and 48 hours respectively.

The result for acid producing from fermented carbohydrate utilized by strain BD3526 is shown in Table 3 and 4.

Table 3 Result of API 50 CHB identification for strain BD3526

| | | | | |
|---|---|---|---|---|
| Control- | D-galactose+ | Glycerinum- | Melibiose+ | Gentiobiose+ |
| Mannitol+ | D-glucose+ | Maltose+ | Saccharose+ | D-lyxose- |
| Erythritol- | D-fructose+ | N-acetyl-glucosamine+ | Xylitol- | D-tagatose- |
| D-arabinose- | D-mannose+ | Amygdalin+ | Raffinose+ | D-fucose- |
| L-arabinose- | L-sorbose- | Arbutin+ | Melezitose- | L-fucose- |
| D-ribose+ | L-rhamnose- | Esculin+ | Lactose+ | D-arabitol- |
| D-xylose+ | Dulcitol- | Salicin+ | Starch+ | L-arabitol- |
| L-xylose- | linositol- | Cellobiose+ | Glycogen+ | Gluconate+ |
| Adonitol- | α-methyl-D-mannoside- | α-methyl-glucoside - | 5-keto-gluconate- | 2-keto-gluconate+ |
| β-methyl-D-xylose+ | Sorbitol- | | | |

"+" represents for producing acid by utilizing carbohydrate, "-" represents for producing no acid.

Table 4 The comparison of acid production for strain BD3526 and similar *Paenibacillus* by means of hydrolyzing carbohydrate

| Strains | 1 | 2 |
|---|---|---|
| Glycerinum | - | w |
| linositol | - | w |
| Gluconate | + | w |

(continued)

| Strains | 1 | 2 |
|---|---|---|
| $\alpha$-methyl-glucoside | - | w |
| 2-keto-gluconate | + | - |
| Trehalose | w | + |
| Note: strain 1 refers to BD3526, and strain 2 refers to *Paenibacillus hunanensis* FeL05$^T$ (ACCC 10718$^T$); W refers to "weak", that is weak positive. | | |

As shown in Table 4, strain BD3526 and strain 2 *Paenibacillus hunanensis* FeL05$^T$ (ACCC 10718$^T$) have a large difference in acid production by means of utilizing carbohydrate, indicating that the two strains belong to different species .

Embodiment 4 the growth characteristics of strain BD3526

1. Growth curve:

1) Adding 30mL of liquid TYC culture medium into each 100mL-Erlenmeyer flask and sealing it with a multilayer (8-12 layers) gauze, autoclaving it at 121°C for 15 minutes;

2) Picking up the fresh cultures from solid TYC agar after a 24-hour incubation and inoculating them into the liquid TYC culture medium mentioned above, shaking culturing them at 30°C and 180r/min for 20-24h to obtain seeds.

3) Transferring the seeds of BD3526 cultured in 2) into the sterilized liquid TYC culture with an inoculum size of 2%(v/v) and mixing them uniformly. Adding 150$\mu$l of the mixed liquor into a costar 96-well sterile microwell plate; arranging 3 parallel controls and taking non-inoculated liquid TYC culture medium as control. Record the $OD_{600nm}$ with an interval of 30min.

2. Growth temperature:

Transferring the seeds of BD3526 cultured in 2) into the sterilized liquid TYC culture with an inoculum size of 2% (v/v) and mixing them uniformly. Incubating them at 4°C, 15°C, 30°C, 37°C, 40°C and 60°C water bath respectively with 3 parallel controls arranged for each temperature gradient, and determining the growth curves thereof 24h and 48h later respectively. The growth temperature range for strain BD3526 is15-40°C, and the preferred temperature is 30°C.

3. NaCl tolerance:

Transferring the seeds of BD3526 cultured in 2%(v/v) into the TYC culture medium with a concentration of sodium chloride of 0.0%, 2.0%, 5.0%, 7.0% and 10.0% respectively and Incubating at 30°C, recording the growth curves 24h and 48h later respectively. The result shows that the NaCl tolerance for strain BD3526 is 10%.

4. pH range for growth

Adjusting the pH value of the sterilized TYC culture medium to 3.0, 4.0, 5.0, 5.5, 6.0, 6.5, 7.0, 8.0, 8.5, 9.0 and 10.0 with sterile HCI and NaOH, inoculating the seeds of BD3526 cultured in 2) with an inoculum size of 2%(v/v) and incubating them at 30°C, recording the growth curves 24h and 48h later respectively. The pH range for growth of strain BD3526 is 5.5-8.5, and the preferred pH value is 6.0.

Embodiment 5 the 16S phylogenetic characteristics for strain BD3526

Obtain the genomic DNA of strain BD3526 in accordance with the operation procedure for Gram-positive bacteria using TIANAMP Bacteria DNA Kit. Determining the absorbance thereof at 230nm, 260nm and 280nm; the A260: A280: A230 thereof is 1: 0.510: 0.445. The purity meets the requirements.
Amplifying the fragment of 16S rDNA for strain BD3526 by using 27F, 1492R-primer. Purifying and then ligating them to the TA cloning vector of pMD19-T Simple Vector, then incubating them at 16°C overnight for transforming into the competent cell of *Escherichia coli* DH5a, smearing them onto the LB agar culture medium with ampicillin and culturing them at 37°C; picking up the positive transformants 16-20 hours later. The positive transformants mentioned above were sent to JIE LI BIOLOGY in Shanghai for sequencing. The result for sequencing was submitted to the database of NCBI and EzTaxon, and the most similar strain found through comparing is *Paenibacillus hunanensis* FeL05$^T$ with a similarity of 96.6%.

Sequence for the primer pair mentioned above is: for 1492R: TACCTTGTTACGACTT (shown in SEQ ID NO.2), and for 27F: AGAGTTTGATCCTGGCTCAG (shown in SEQ ID NO.3).

The result of gene sequencing for 16S rRNA of strain BD3526 is shown in SEQ ID NO.1.

The sequence of 16S rRNA mentioned above is compared by software of CLUSTAL_X program (version 1.83) and the phylogenic tree is drawn by MEGA version 4.0.2. software. Using neiglibor-joining for calculation with maximum-parsimony and maximum-likelihood for proof calculation, the bootstrap is set as 1000 cycles and the result is shown in FIG. 2. As shown in FIG. 2, through the analysis on phylogenetic tree of 16S rRNA gene, strain BD3526 shall be classified as the cluster of *Paenibacillus hunanensis.* However, the similarity between strain BD3526 and the type strain of *Paenibacillus hunanensis* is 96.6%. Meanwhile, 97% is the threshold for similarity between the 16S rRNAs of different strains in the same species, moreover, lots of literatures have proven that for some bacteria with the similarity over 99% on gene sequence for 16S rRNA are still classified as different strains; So that the strain BD3526 is likely to be a new species belong to genus *Paenibacillus* and thus other physiological and biochemical indexes should to be determined.

Embodiment 6 the characteristics on total cellular fatty acid content for strain BD3526

Determination of total cellular fatty acid content of strain BD3526.

Preparing the following solutions: I , dissolving 45g of sodium hydroxide with 150ml of methanol and 150ml of distilled water; II, dissolving 190ml of concentrated hydrochloric acid and 275ml of methanol with 135ml of distilled water; III, mixing 200ml of normal hexane with 200ml of ethyl ether uniformity; IV, dissolving 10.8g of sodium hydroxide with 900ml of distilled water; V, saturating sodium chloride solution.

1) Adding the bacterial culture in a moderate amount into a 8ml-screwed glass tube with 1ml of solution I added. Tightening the screw cap and bathing it in boiling water for 5min, take it out for a 5-10S-shaking and then continue the bathing for 25min;

2) Once the sample tube is cooled, adding 2ml of solution II into it and covering it tightly for shaking, then bathing it for 10min at 80$\pm$1°C with an accurate control and cooling it with ice-bath;

3) Adding 1.25ml of solution III into the solution mentioned above and rapidly shaking it for about 10min to discard the lower aqueous phase;

4) Adding 3ml of solution IV and 0.1-0.2ml of solution V into the remaining organic phase and rapidly shaking it for about 5min, taking two thirds of the upper organic phase and adding it into the sample bottle for chromatography.

The HP6890 gas chromatograph, equipped with split / splitless inlet, flame ionization detector (FID) and HP gas chromatograph chemstation; the chromatographic column is Ultra-2 column with length of 25m, inner diameter of 0.2mm, and liquid film thickness of 0.33$\mu$m; the furnace temperature is two step programmed temperature: initial temperature is 170°C, raised to 260°C in rate of 5°C/min and then raised into 310°C in rate of 40°C/min and maintain it for 1.5min; the temperature at inlet is 250°C and the carrier gas is oxygen with a flow rate of 0.5ml/min in split mode and the split ratio is 100: 1, the sample size is 2$\mu$l; the temperature for test is 300°C, the flow rate of hydrogen is 30ml/min, the flow rate of air is 216ml/min and the flow rate of supplemental gas (nitrogen) is 30ml/min.

The result shows that the main cellular fatty acids of strain BD3526 are anteiso saturated fatty acid $C_{15:0}$, anteiso heptadecenoic saturated fatty acid, anteiso hexadecanoyl saturated fatty acid,and hexadecanoyl saturated fatty acid, wherein the percentage contents are 59.02%, 11.09% and 7.66% respectively. The main fatty acid in accordance with the *Paenibacillus* is anteiso saturated fatty acid $C_{15:0}$, and both type and content of fatty acid thereof are different from the similar strains, therefore the strain is further judged as a novel member belonging to the family of *Paenibacillus.*

Embodiment 7 the characteristics on G+C mol% content for strain BD3526

Determination of G+C mol% content of genomic DNA for strain BD3526.

The melting temperature (Tm) method is adopted with *E.coli* K12, AS 1.365 as reference control, the device used is the Lambda35 UV/VIS Spectrometer of Perkin/Elmer; the temperature is controled by PTP-1 temperature digital controller.

The steps are as follows:

1) Diluting the sample DNA to be tested with 0.1×SSC into an $OD_{260nm}$ value ranges within 0.3-0.4;

2) Recording the $OD_{260nm}$ value at 25°C firstly and then setting the temperature raising procedure to raise the temperature from 65°C to 95°C at arate of 1°C per minute;

3) A raising OD value indicates the beginning of denaturation, record the temperature of cuvette and the OD value until the OD value stays stable (indicating the completion of denaturation);

4) Obtaining the denaturation temperature (Tm) based on the thermal denaturation curve and calculating the G+Cmol% content.

The calculation formula in 0.1×SSC solution is:

$$G{+}C\ mol\% = G{+}C\ mol\%AS_{1.365}+2.08\ (Tm_{unknown}-Tm_{AS1.365})$$

The Tm of *E. coli K12,* $AS_{1.365}$ determined in the test is 75.810°C, the Tm value and G+C mol% for strain to be tested.

The result of G+C mol% for strain BD3526 is shown in Table 5.

Table 5 the G+C mol% content for strain BD3526

| Strain number | Tm value (°C) | G+C mol% |
|---|---|---|
| Control *E coli* K12 | 75.810 | - |
| BD3526 | 74.024 | 47.48 |

The G+C mol% for strain BD3526 is 47.48%, and the G+C mol% for *Paenibacillus hunanensis* FeL05$^T$ (ACCC 10718$^T$=CGMCC 1.8907$^T$=DSM 22170$^T$) is 53.3% (the difference between them is greater than 5%). The G+C content of *Paenibacillus* ranges within 45-54 mol%. According to the "Common Bacteria Identification System Manual"(Dong Xiuzhu, Cai Miaoying), the G+C content of *Paenibacillus* ranges within 45-54 mol%; the difference between G+C mol% of the two strains is greater than 5%, therefore the two strains can be judged as different species (the judgment mentioned above is appropriate even when other characters are similar). Therefore, the strain BD3526 is classified as a novel member belongs to *Paenibacillus* sp. and different from its most-similar strain *Paenibacillus hunanensis* FeL05$^T$ (ACCC 10718$^T$=CGMCC 1.8907$^T$=DSM 22170$^T$).

Embodiment 8 the homologous hybridization experiment for strain BD3526

The hybridization experiment between strain BD3526 and the strain with the closest genetic relationship and the type strain of *Paenibacillus.*

Conducting the DNA-DNA hybridization experiment between strain BD3526 and *Paenibacillus hunanensis* FeL05$^T$ (ACCC 10718$^T$= CGMCC 1.8907$^T$ = DSM 22170$^T$) with the most close genetic relationship and the type strain *Paenibacillus polymyxa* ATCC 842$^T$ (=CGMCC 1.4261$^T$=DSM 36$^T$=KCTC 3858$^T$) for *Paenibacillus* with reference to the result of 16S rRNA.

The method of renaturation rate in liquid phase is adopted, and the device used is the Perkin Elmer Lambda35 UV/VIS Spectrophotometer. The temperature is controlled by PTP-1 Peltier System. The steps are as follows:

1) DNA sample processing: the DNA sample extracted as in Embodiment 5 mentioned above. The concentration of DNA sample was adjusted to $OD_{260}$nm 2.0. Before the experiment, the sample was cooled with ice-bath and sonicate at a power level of 40W for 24 minutes to break the DNA into fragments of 2-5 X$10^5$ daltons. (Setting as: sonicating for 3 seconds / pausing for 3 seconds).

2) Preparing the DNA sample (A,B) to be tested with the $OD_{260}$nm value thereof ranges within 1.8-2.0 by using 0.1 ×SSC respectively, the $OD_{260}$ nm value for both of them shall be consistent (accurate to 0.001);

3) Entering the UV Winlab program (manufacturer: Perkin Elmer) and the window for the method will appear. Selecting the "Time-Driven" (TD) method and setting the proper parameters for determination with the setting page of "Timed. Inst. Sample.". The wavelength for determination is 260 nm and the total time for determination is set for 30 minutes. Calculating the optimal renaturation temperature (TOR) on the basis of the determined G+C mol%, and keeping the temperature of cuvette at the optimal renaturation temperature steadily. In 2x SSC reaction liquid, the formula for optimal renaturation temperature is: TOR = 0.51 $\times$ (G+C) mol% + 47.

4) Adding 400μl of the DNA samples of two strains into two centrifugal tubes respectively and then adding 200μl of the DNA samples of two strains into the same centrifugal tube to form the mixed sample;

5) Before testing, the single DNA sample and mixed DNA sample shall be denatured for 15min at 100°C with PTP-1 temperature-control system (manufacturer: Perkin Elmer) and then cooled to the optimal renaturation temperature. Record the $OD_{260}$nm value and stop the reading after a 30-min reaction. The temperature of samples shall not be lower than TOR during the whole process. Finally, a straight line representing a decreasing absorbance value with time will be obtained;

6) Using software UV Winlab, selecting "Slope" in "Algorithm" column thereof to obtain the renaturation rate (V), namely slope ("V" usually represents the reduced amount for absorbance per minute);

7) Calculating the homogenous hybridization rate in accordance with the formula.

$$\text{Homogenous hybridization rate (H) \%} = 4Vm — (Va+Vb) /2 \sqrt{VaVb} \times 100\%$$

The result for DNA-DNA hybridization is as follows:

BD3526/ *Paenibacillus hunanensis* FeL05$^T$ (repeat for three times):

H%=39.82% (I);

H%=41.60% (II);

H%=42.10% (IV).

BD3526/ *Paenibacillus polymyxa* ATCC 842$^T$ (repeat for three times):

H%=41.62% (I);

H%=46.60% (II);

H%=48.60% (III).

The result shows that the DNA-homology between strain BD3526 and *Paenibacillus hunanensis* FeL05$^T$ (ACCC 10718$^T$=CGMCC 1.8907$^T$ =DSM 22170$^T$) is 39.82-42.10%, and for the type strain *Paenibacillus polymyxa* ATCC 842$^T$ (=CGMCC 1.4261$^T$=DSM 36$^T$=KCTC 3858$^t$) of *Paenibacillus,* the DNA-homology is 41.62-48.60%. Reference to "Bergey's Manual of Determinative Bacteriology", under the optimum conditions, a DNA-homology greater than 70% belongings to the same species and that greater than 20% belonging to the same genus. Combining with the data of Embodiment 2, 3, 4, 5 and 6, strain BD3526 is classified into a new species of *Paenibacillus.* The taxonomic status of this strain is *Paenibacillus* spp., and it is likely to be named as *Paenibacillus bovis* sp. nov. in accordance with the naming method of International Committee Systematic Bacteriology, while strain BD3526 is selected as the type strain of this species.

Embodiment 9 determination for MCA of the fermentation broth extracts of strain BD3526

Inoculating the new species *Paenibacillus* spp. BD3526 (*Paenibacillus bovis* sp. nov.) into the TYC broth for a 16h-incubation at 35°C and 180r/min to obtain inoculum seeds. Then inoculating the seeds into a 250mL-Erlenmeyer flask which containing 20mL of 1%-wheat bran broth with an inoculum size of 9% (v/v) and mixing them uniformity; shaking incubating them at 20°C and 300r/min for 18h. Take 5mL of fermented wheat bran broth for a centrifugation at 4°C and 9000r/min to obtain the supernatant, namely the crude MCE of BD3526.

The prepared 500μl-crude MCE is used for the milk clotting experiment in accordance with the following method, the determination method for milk clotting activity (MCA) comprises the following steps:
Preparing the 10 % (w/v) reconstructed skim milk solution containing 10mmol/L of $CaCl_2$ with pH value of 6.0. Preincubating it at 35°C water-bath for 10min and then adding 500μl of crude MCE into the test tube containing 10mL-skim milk solution, taking out the sample and inclining it at 45° every 15s to observe the texture status thereof. The time for forming discontinuous particles shall be regarded as the clotting time. One Soxhlet unite (SU) is defined as the amount of enzyme for clotting 1mL-skim milk in 40 minutes at 35°C.

$$MCA\ (SU/mL) = \frac{2400 \times V_S}{T \times V_E} \times D$$

T - clotting time (s)

$V_s$ - volume of substrate (mL)

$V_E$ - volume of enzyme extracts (mL)

D - dilution ratio

The determination method for proteolytic activity (PA) is as follows:
Proteolytic activity is determined by Folin-Ciocalteu phenol method, using casein substrate. Adding 1mL of the fermented supernatant into 5mL of 12g/L-casein solution prepared with phosphate solution with a concentration of 0.05mol/L and pH value of 6.0 and mixing them uniformity. Incubating them at 35°C water-bath for 10 minutes and adding 0.44mol/L TCA to terminate the reaction; then centrifuging them at -4°C and 10000r/min to obtain the supernatant. Adding 5 mL of 0.28mol/L-NaOH solution and 1.5 mL of Folin-Ciocalteu phenol reagent (diluted with deioned water in the ratio of 1:2) into 2mL of the supernatant. Incubating them at 35°C water-bath for 15 minutes and then determining the $A_{660nm}$. Preparing the tyrosine solution with the concentration of 100μl/mL and determining the $A_{660nm}$ of tyrosine solutions with the concentration of 0, 20, 40, 60, 80 and 100μl/mL respectively in accordance with the Folin-Ciocalteu phenol method and establishing the standard curve. One proteolytic activity unit is defined as the amount of enzyme required for releasing 1μmol of tyrosine in 1min.

The determined experimental result shows that the clotting time for *Paenibacillus* spp. BD3526 (CGMCC No.8333) is 57.67 ±2.05 seconds, and the MCA of the fermentation broth extracts for this strain calculated in accordance with the method mentioned above is 833.43±29.98SU/mL; the determined proteolytic activity of this fermentation broth extracts is 1.53±0.18U/mL, and the ratio of MCA/PA is 544.72, the form of curd prepared with the extracts of *Paenibacillus* spp. BD3526 (CGMCC No.8333) is shown in FIG. 3.

Embodiment 10 determination for MCA of the fermentation broth extracts of strain BD3526

Inoculating the *Paenibacillus* CGMCC No.8333 according to Embodiment 1 into the TYC culture medium for a 20h-incubation at 25°C and 180r/min to obtain seeds; inoculating the seeds into a 250mL-Erlenmeyer flask containing 50mL of 3%-wheat bran broth with an inoculum size of 5% (v/v) and mixing them uniformity; then Incubating them for 28h at 30°C and 200r/min in shaking flask.

After incubating, taking 5mL of cultured wheat bran broth for a centrifugation at 0°C and 9000r/min to obtain the supernatant, namely the crude extract containing MCEs. Diluting the prepared 500μL-crude enzyme extracts for 5-fold (D=5) and determining the MCA and PA in accordance with the method in Embodiment 9.

The determined clotting time is 78.33±1.25 seconds, the calculated milk clotting activity of the crude enzyme extracts is 3064.60±48.51SU/mL; and the determined proteolytic activity is 5.21 ±0.47U/mL, the ratio of MCA/PA is 588.21.

Embodiment 11 determination for MCA of the fermentation broth extracts of *Paenibacillus* CGMCC No.8333

Inoculating the *Paenibacillus* CGMCC No.8333 according to Embodiment 1 into the TYC culture medium for a 20h-culture at 30°C and 180r/min to obtain seeds; inoculating the seeds into a 250mL-Erlenmeyer flask containing 100mL of 14%-wheat bran broth with an inoculum size of 1%(v/v) and mixing them uniformity; then incubating them for 120h at 30°C and 200r/min in shaking flask.

After incubation, taking 5mL of cultured wheat bran broth for a centrifugation at 4°C and 9000r/min to obtain the supernatant, namely the crude extract containing MCE.

Determining the MCA and PA with the prepared 500μL-crude enzyme extracts in accordance with the method in Embodiment 9.

The determined clotting time is 67.33±1.63 seconds, the calculated milk clotting activity of the crude enzyme extracts is 716.84±17.48SU/mL; and the determined proteolytic activity is 1.39 ±0.29U/mL, the ratio of MCA/PA is 515.71.

Embodiment 12 determination for milk-clotting activity of the fermentation broth extracts of *Paenibacillus* CGMCC No.8333

Inoculating the *Paenibacillus* CGMCC No.8333 according to Embodiment 1 into the TYC culture medium for a 20h-culture at 30°C and 180r/min to obtain seeds; inoculating the seeds into a 250mL-Erlenmeyer flask containing 30mL of 3%-wheat bran broth with an inoculum size of 5% (v/v) and mixing them uniformity; then incubating them for 20h at 30°C and 300r/min in shaking flask. After incubation, taking 5mL of cultured wheat bran broth for a centrifugation at 4°C and 9000r/min to obtain the supernatant, namely the crude extract containing MCEs.

Diluting the prepared 500μL-crude enzyme extracts for 10-fold (D=10) and determining the MCA and PA in accordance with the method in Embodiment 9.

The determined clotting time is 74.33±3.26 seconds, the calculated milk clotting activity of the crude enzyme extracts is 6469.72±280.65SU/mL; and the determined proteolytic activity is 10.54 ±0.65U/mL, the ratio of MCA/PA is 613.82.

Embodiment 13 determination for milk-clotting activity of the fermentation broth extracts of *Paenibacillus* CGMCC No.8333

Inoculating the *Paenibacillus* CGMCC No.8333 according to Embodiment 1 into the TYC culture medium for a 18h-culture at 30°C and 180r/min to obtain seeds; inoculating the seeds into a 250mL-Erlenmeyer flask containing 20mL of 3%-wheat bran broth with an inoculum size of 3%(v/v) and mixing them uniformity; then incubating them for 18h at 40°C and 300r/min in shaking flask. After incubation, taking 5mL of cultured wheat bran broth for a centrifugation at 4°C and 14000r/min to obtain the supernatant, namely the crude extract containing MCE.

Determining the MCA and PA with the prepared 500μL-crude enzyme extracts in accordance with the method in Embodiment 9.

The determined clotting time is 3720±86.41 seconds, the calculated milk clotting activity of the crude enzyme extracts is 12.91±0.30SU/mL; and the determined proteolytic activity is 0.04 ±0.02U/mL, the ratio of MCA/PA is 322.75.

Embodiment 14 determination for Milk-clotting activity of the fermentation broth extracts of *Paenibacillus* CGMCC No.8333

Inoculating the *Paenibacillus* CGMCC No.8333 according to Embodiment 1 into the TYC culture medium for a 18h-culture at 35°C and 180r/min to obtain seeds; inoculating the seeds into a 250mL-Erlenmeyer flask containing 50mL of 10%-wheat bran broth with an inoculum size of 7%(v/v) and mixing them uniformity; then incubating them for 48h at 35°C and 180r/min in shaking flask. After incubation, taking 5mL of cultured wheat bran broth for a centrifugation at 4°C and 14000r/min to obtain the supernatant, namely the crude extract containing MCE.

Diluting the prepared 500μL-crude enzyme extracts for 5-fold (D=5) and determining the MCA and PA in accordance with the method in Embodiment 9.

The determined clotting time is 240.33±2.49 seconds, the calculated milk clotting activity of the crude enzyme extracts is 998.72±10.41 SU/mL; and the determined proteolytic activity is 1.67 ±0.27U/mL, the ratio of MCA/PA is 598.03.

Embodiment 15 determination for milk-clotting activity of the fermentation broth extracts of *Paenibacillus* CGMCC No.8333

Inoculating the *Paenibacillus* CGMCC No.8333 according to Embodiment 1 into the TYC culture medium for an 18h-culture at 30°C and 140r/min to obtain seeds; inoculating the seeds into a 250mL-Erlenmeyer flask containing 30mL of 3%-wheat bran broth with an inoculum size of 5% (v/v) and mixing them uniformity; then, incubating them for 20h at 25°C and 300r/min in shaking flask. After incubation, taking 5mL of cultured wheat bran broth for a centrifugation at 4°C and 14000r/min to obtain the supernatant, namely the crude extract containing MCE.

Diluting the prepared 500μL-crude enzyme extracts for 10-fold (D=10) and determining the MCA and PA in accordance with the method in Embodiment 9.

The determined clotting time is 115.67±2.49 seconds, the calculated milk clotting activity of the crude enzyme extracts is 4151.77±88.83SU/mL; and the determined proteolytic activity is 6.89 ±0.95U/mL, the ratio of MCA/PA is 602.58.

Contrastive embodiment 1 the determination of milk clotting activity of the crude enzyme extracts with different microbial origins

Conducting the clotting experiment on the prepared 500μl-crude enzyme extracts with other microbial origins in accordance with the method in Embodiment 2, the milk clotting activity data of the obtained crude enzyme extracts are show in Table 6 below:

Table 6 Determination result of milk clotting activity for crude enzyme extracts with other microbial origins

| Microorganisms | Activity for crude enzyme extracts |
|---|---|
| *Mucor rouxii*[1] | 1.90 SU/mL |
| *Penicillium oxalicum*[2] | 16.8 SU/mg |
| *Nocardiopsis* spp.[3] | 4.7 SU/mL |
| *Bacillus amyloliquefaciens* D4[4] | 1000 SU/mL |
| *Bacillus subtilis* (natto) Takahashi[5] | 685.7 SU/mL |
| *Bacillus subtilis* B1 [6] | 1129.05 SU/mL |
| *Bacillus subtilis* YB-3[7] | 200 SU/mL |
| *Bacillus sphaericus* NRC 24[8] | 1212 SU/mL |

For information on the *Mucor rouxii* according to Table 6, please refer to [1]: Yu P J, Chou C C. Factors affecting the growth and production of milk clotting enzyme by Amylomyces rouxii in rice liquid medium[J]. Food Technology and Biotechnology, 2005, 43(3): 283-288.;

For information on the *Penicillium oxalicum* according to Table 6, please refer to [2]: Hashem MA. Purification and properties of a milk clotting enzyme produced by Penicillium oxalicum[J]. Bioresource Technology, 2000, 75(3): 219 - 222.;

For information on the *Nocardiopsis* spp. according to Table 6, please refer to [3]: Cavalcanti MTH, Martinez CR, Furtado VC, Neto BB, Teixeira MF, Lima Filho JL, Porto ALF.Milk-clotting protease production by Nocardiopsis sp. in an inexpensive medium[J] .World Journal of Microbiology and Biotechnology, 2005, 21(2): 151-154.;

For information on the *Bacillus amyloliquefaciens* D4 according to Table 6, please refer to [4]: He X, Zhang W, Ren F, Gan B, Guo H. Screening fermentation parameters of the milk-clotting enzyme produced by newly isolated Bacillus amyloliquefaciens D4 from the Tibetan Plateau in China[J]. Annals of Microbiology, 2012, 62(1): 357-365.;

For information on the Bacillus subtilis (natto) Takahashi according to Table 6, please refer to [5]: Shieh C J, Phan Thi LA, Shih I L. Milk-clotting enzymes produced by culture of Bacillus subtilis natto [J].Biochemical Engineering Journal, 2009, 43 (1): 85-91.;

For information on the *Bacillus subtilis* B1 according to Table 6, please refer to [6]: Ding Z Y, Liu S P, Gu Z H, Zhang L, Zhang K C, Shi G Y. Production of milk-clotting enzyme by Bacillus subtilis B1 from wheat bran[J], African Journal of Biotechnology, 2011, 10(46): 9370-9378.;

For information on the *Bacillus subtilis* YB-3 according to Table 6, please refer to [7]: Li Y, Liang S, Zhi D, Chen P, Su F, Li H. Purification and characterization of Bacillus subtilis milk-clotting enzyme from Tibet Plateau and its potential use in yak dairy industry [J]. European Food Research and Technology, 2012, 234(4): 733-741.;

For information on the *Bacillus sphaericus* NRC 24 according to Table 6, please refer to [8]: El-Bendary M A, Moharam M E, Ali T H. Purification and Characterization of Milk Clotting Enzyme Produced by Bacillus sphaericus [J]. Journal of Applied Sciences Research, 2007, 3(8): 695-699.

The experimental result above shows that the milk clotting activity of the crude enzyme extracts prepared with *Paenibacillus* spp. BD3526 (*Paenibacillus bovis* sp. nov.) CGMCC No.8333 is several times or even hundred times higher than that of the crude enzyme extracts with other microbial origins, filling the large gap in field of MCE-preparation.

Contrastive embodiment 2 preparing the fermentation broth extracts of *Paenibacillus* CGMCC No.8333 by using different culture media

Inoculating the *Paenibacillus* CGMCC No.8333 according to Embodiment 1 into the TYC culture medium for an 18h-culture at 30°C and 180r/min to obtain seeds; inoculating the seeds of the strain into a 250mL-Erlenmeyer flask containing 50mL of 3%-wheat bran broth and a 250mL-Erlenmeyer flask containing 50mL LB broth with an inoculum size of 3%(v/v) respectively and mixing them uniformity; then incubating them for 30h at 30°C and 100r/min in shaking flask. After incubation, take 5mL of the cultured wheat bran broth and 5mL of the LB broth respectively for a centrifugation at 4°C and 9000r/min to obtain the supernatant, namely the crude extract containing MCEs.

Diluting the prepared 500μL-crude enzyme extracts of the wheat bran broth for 5-fold (D=5) and not diluting the crude enzyme extracts of the LB broth (D=1), then determining the MCA and PA in accordance with the method in Embodiment 9.

The determined clotting time for crude enzyme extracts of the wheat bran broth is 77.67±2.52 seconds, the calculated milk clotting activity of the crude enzyme extracts is 3092.31±100.88SU/mL; and the determined proteolytic activity is 5.23 ±0.51U/mL, the ratio of MCA/PA is 591.26.

The determined clotting time for crude enzyme extracts of the LB broth is 3630.67±137.48 seconds, the calculated milk clotting activity of the crude enzyme extracts is 13.24±0.51SU/mL; and the determined proteolytic activity is 0.03 ±0.001U/mL, the ratio of MCA/PA is 441.33. Thus, the milk clotting activity of the fermentation broth extracts obtained from the crude enzyme extracts prepared with wheat bran broth is 233.56 times higher than that of the LB culture medium.

## Claims

1. A preparation method for preparing fermentation broth extracts having milk-clotting activity, using a wheat bran broth, **characterized in that** the method for preparing comprising the steps of: obtaining the fermentation broth by shaking culture Paenibacillus (Paenibacillus bovis sp. nov.) CGMCC No.8333 at 15°C-40°C for 18-120 hours in said broth, and obtaining the extracts by taking out the supernatant after centrifuging the resulting fermentation broth.

2. The preparation method according to Claim 1, **characterized in that** the components of the wheat bran broth comprise wheat bran and water, wherein the content of wheat bran ranges within 1%-14%, and the percentages refer to the mass percentages.

3. The preparation method according to Claim 1 or 2, **characterized in that** the rotation speed for the shaking culture ranges within 100 r/min-300 r/min.

4. The preparation method according to Claim 3, **characterized in that** the content of wheat bran of the wheat bran broth in mass percentage ranges within 1%-10%, the rotation speed for the shaking culture ranges within 140 r/min-300 r/min, and the duration for the shaking culture ranges within 20-48 hours.

5. The preparation method according to at least one of Claims 1-4, **characterized in that** the temperature for the centrifuging ranges within 0°C-4°C, and the speed for the centrifuging ranges within 9000r/min-14000r/min.

6. The preparation method according to at least one of Claims 1-5, **characterized in that** the preparation method also comprises the step of activation of a strain, and the step of activation of the strain is: inoculating the Paenibacillus CGMCC No.8333 into the TYC broth for activating culture for 18-48 hours at 25°C-35°C to obtain the seeds; and inoculating the activated strain into the wheat bran broth which comprises wheat bran ranging in a mass percentage within 1%-14% in an inoculum size of 1-9% by volume for shaking culture.

7. The preparation method according to Claim 6, **characterized in that** the duration for the activating culture ranges within 18-48 hours, the rotation speed for the shaking culture ranges within 140-300 r/min, the temperature for the shaking culture ranges within 25°C-35°C; and the duration for the shaking culture ranges within 18-120 hours.

8. The preparation method according to at least one of Claims 1-7, **characterized in that** the preparation method also comprises a step of refining and drying the supernatant obtained, wherein the step of refining and drying refers to vacuum freeze drying, and the temperature for the vacuum freeze drying ranges within -44°C to -40°C.

## Patentansprüche

1. Herstellungsverfahren zur Herstellung von Fermentationsbrühe-Extrakten mit Milchgerinnungsaktivität unter Verwendung einer Weizenkleie-Brühe, **dadurch gekennzeichnet, dass** das Herstellungsverfahren folgende Schritte umfasst: Erhalten der Fermentationsbrühe durch Schüttelkultur von Paenibacillus (*Paenibacillus* bovis sp. nov.) CGMCC Nr. 8333 bei 15°C-40°C für 18-120 Stunden in der Brühe, und Erhalten der Extrakte durch Herausnehmen des Überstandes nach der Zentrifugation der resultierenden Fermentationsbrühe.

2. Herstellungsverfahren nach Anspruch 1, **dadurch gekennzeichnet, dass** die Bestandteile der Weizenkleie-Brühe Weizenkleie und Wasser umfassen, wobei der Gehalt an Weizenkleie im Bereich von 1%-14% liegt und sich die Prozentsätze auf die Massenanteile beziehen.

3. Herstellungsverfahren nach Anspruch 1 oder 2, **dadurch gekennzeichnet, dass** die Drehzahl für die Schüttelkultur im Bereich von 100 U/min-300 U/min liegt.

4. Herstellungsverfahren nach Anspruch 3, **dadurch gekennzeichnet, dass** der Gehalt an Weizenkleie der Weizenkleie-Brühe in Massenanteile im Bereich von 1%-10% liegt, die Drehzahl für die Schüttelkultur im Bereich von 140 U/min-300 U/min liegt, und die Dauer für die Schüttelkultur im Bereich von 20-48 Stunden liegt.

5. Herstellungsverfahren nach mindestens einem der Ansprüche 1-4, **dadurch gekennzeichnet, dass** die Temperatur für die Zentrifugation im Bereich von 0°C-4°C liegt und die Drehzahl für die Zentrifugation im Bereich von 9000 U/min-14000 U/min liegt.

6. Herstellungsverfahren nach mindestens einem der Ansprüche 1-5, **dadurch gekennzeichnet, dass** das Herstellungsverfahren auch den Schritt der Aktivierung eines Stammes umfasst, und der Schritt der Aktivierung des Stammes folgendes ist: Inokulieren des Paenibacillus CGMCC Nr. 8333 in die TYC-Brühe für die Aktivierungskultur für 18-48 Stunden bei 25°C-35°C, um die Inokula zu erhalten; und Inokulieren des aktivierten Stammes in die Weizenkleie-Brühe, die Weizenkleie in einem Massenanteilbereich von 1%-14% umfasst, in einer Inokulumgröße von 1-9 Vol. % für die Schüttelkultur.

7. Herstellungsverfahren nach Anspruch 6, **dadurch gekennzeichnet, dass** die Dauer für die Aktivierungskultur im Bereich von 18-48 Stunden liegt, die Drehzahl für die Schüttelkultur im Bereich von 140-300 U/min liegt, die Temperatur für die Schüttelkultur im Bereich von 25°C-35°C liegt; und die Dauer für die Schüttelkultur im Bereich von 18-120 Stunden liegt.

8. Herstellungsverfahren nach mindestens einem der Ansprüche 1-7, **dadurch gekennzeichnet, dass** das Herstellungsverfahren auch einen Schritt zum Verfeinern und Trocknen des erhaltenen Überstandes umfasst, wobei sich der Schritt zum Verfeinern und Trocknen auf die Vakuumgefriertrocknung bezieht, und die Temperatur für die Vakuumgefriertrocknung im Bereich von - 44°C bis -40°C liegt.

## Revendications

1. Procédé de préparation pour la préparation d'extraits de bouillon de fermentation ayant une activité de coagulation du lait, en utilisant du bouillon de son de blé, **caractérisé en ce que** le procédé pour la préparation comprend les étapes consistant à : obtenir le bouillon de fermentation en agitant la culture *Paenibacillus* (*Paenibacillus bovis sp. nov.*) CGMCC N° 8333 à 15 °C-40 °C pendant 18-120 heures dans ledit bouillon, et obtenir les extraits en retirant le surnageant après la centrifugation du bouillon de fermentation obtenu.

2. Procédé de préparation selon la revendication 1, **caractérisé en ce que** les composants du bouillon de son de blé comprennent du son de blé et de l'eau, dans lequel la teneur en son de blé est comprise entre 1 %-14 %, et les pourcentages font référence aux pourcentages en masse.

3. Procédé de préparation selon la revendication 1 ou 2, **caractérisé en ce que** la vitesse de rotation pour la culture agitée est comprise entre 100 t/min-300 t/min.

4. Procédé de préparation selon la revendication 3, **caractérisé en ce que** la teneur en son de blé du bouillon de son de blé en pourcentage en masse est comprise entre 1 %-10 %, la vitesse de rotation pour la culture agitée est comprise entre 140 t/min-300 t/min, et la durée pour la culture agitée est comprise entre 20-48 heures.

5. Procédé de préparation selon au moins l'une des revendications 1-4, **caractérisé en ce que** la température pour la centrifugation est comprise entre 0 °C-4 °C, et la vitesse pour la centrifugation est comprise entre 9 000 t/min-14 000 t/min.

6. Procédé de préparation selon au moins l'une des revendications 1-5, **caractérisé en ce que** le procédé de préparation comprend également l'étape d'activation d'une souche, et l'étape d'activation de la souche est : inoculer le *Paenibacillus* CGMCC N° 8333 dans le bouillon TYC pour activer la culture pendant 18-48 heures à 25 °C-35 °C pour obtenir les graines ; et inoculer la souche activée dans le bouillon de son de blé qui comprend le son de blé dans un pourcentage en masse compris entre 1 %-14 % dans une taille de l'inoculum de 1-9 % en volume pour la culture agitée.

7. Procédé de préparation selon la revendication 6, **caractérisé en ce que** la durée pour la culture activée est comprise entre 18-48 heures, la vitesse de rotation pour la culture agitée est comprise entre 140-300 t/min, la température pour la culture agitée est comprise entre 25 °C-35 °C ; et la durée pour la culture activée est comprise entre 18-120 heures.

8. Procédé de préparation selon au moins l'une des revendications 1-7, **caractérisé en ce que** le procédé de préparation comprend également une étape de raffinage et de séchage du surnageant obtenu dans lequel l'étape de raffinage et de séchage fait référence à la lyophilisation sous vide, et la température pour la lyophilisation sous vide est comprise entre -44 °C à -40 °C.

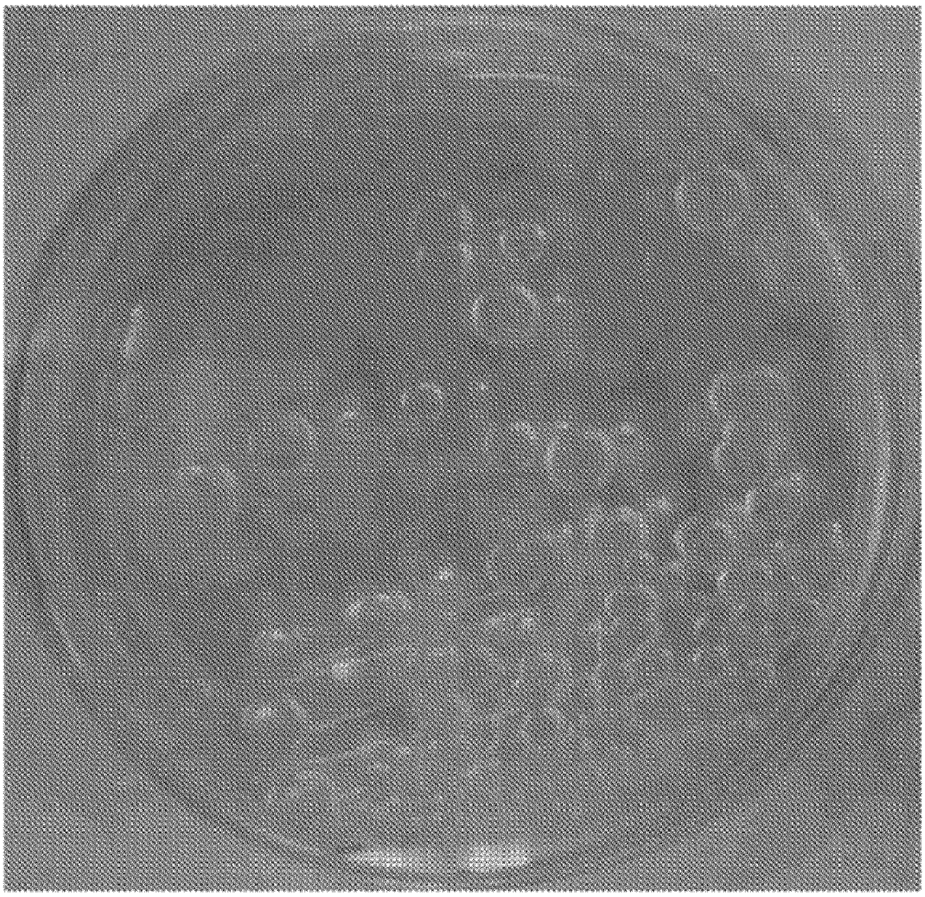

FIG. 1

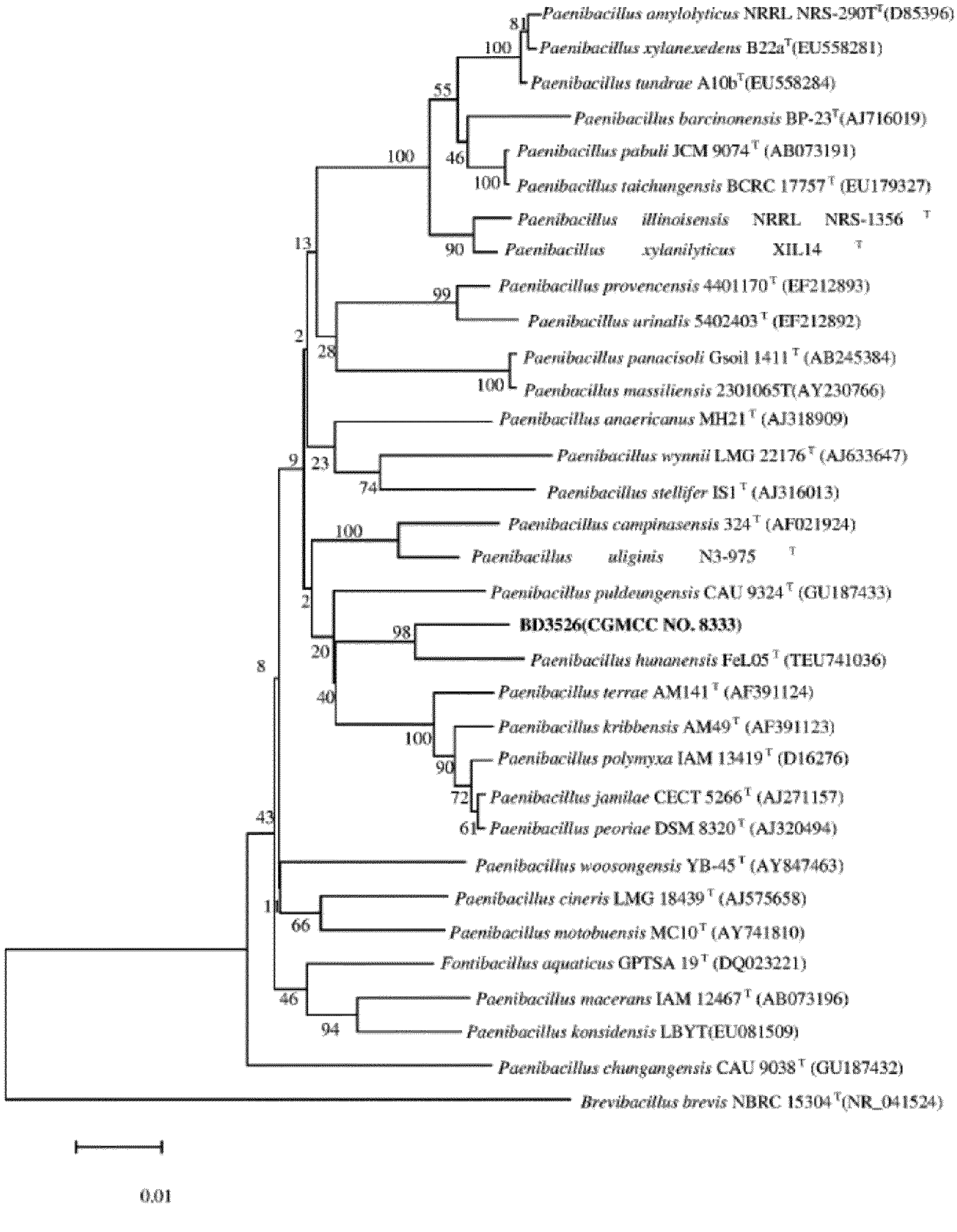
Paenibacillus amylolyticus NRRL NRS-290T T (D85396)
Paenibacillus xylanexedens B22a T (EU558281)
Paenibacillus tundrae A10b T (EU558284)
Paenibacillus barcinonensis BP-23 T (AJ716019)
Paenibacillus pabuli JCM 9074 T (AB073191)
Paenibacillus taichungensis BCRC 17757 T (EU179327)
Paenibacillus illinoisensis NRRL NRS-1356 T
Paenibacillus xylanilyticus XIL14 T
Paenibacillus provencensis 4401170 T (EF212893)
Paenibacillus urinalis 5402403 T (EF212892)
Paenibacillus panacisoli Gsoil 1411 T (AB245384)
Paenbacillus massiliensis 2301065T(AY230766)
Paenibacillus anaericanus MH21 T (AJ318909)
Paenibacillus wynnii LMG 22176 T (AJ633647)
Paenibacillus stellifer IS1 T (AJ316013)
Paenibacillus campinasensis 324 T (AF021924)
Paenibacillus uliginis N3-975 T
Paenibacillus puldeungensis CAU 9324 T (GU187433)
BD3526(CGMCC NO. 8333)
Paenibacillus hunanensis FeL05 T (TEU741036)
Paenibacillus terrae AM141 T (AF391124)
Paenibacillus kribbensis AM49 T (AF391123)
Paenibacillus polymyxa IAM 13419 T (D16276)
Paenibacillus jamilae CECT 5266 T (AJ271157)
Paenibacillus peoriae DSM 8320 T (AJ320494)
Paenibacillus woosongensis YB-45 T (AY847463)
Paenibacillus cineris LMG 18439 T (AJ575658)
Paenibacillus motobuensis MC10 T (AY741810)
Fontibacillus aquaticus GPTSA 19 T (DQ023221)
Paenibacillus macerans IAM 12467 T (AB073196)
Paenibacillus konsidensis LBYT(EU081509)
Paenibacillus chungangensis CAU 9038 T (GU187432)
Brevibacillus brevis NBRC 15304 T (NR_041524)
81
100
55
46
100
100
90
13
99
2
28
100
9
23
74
100
2
98
20
8
40
100
90
72
61
43
1
66
46
94
0.01

FIG. 2

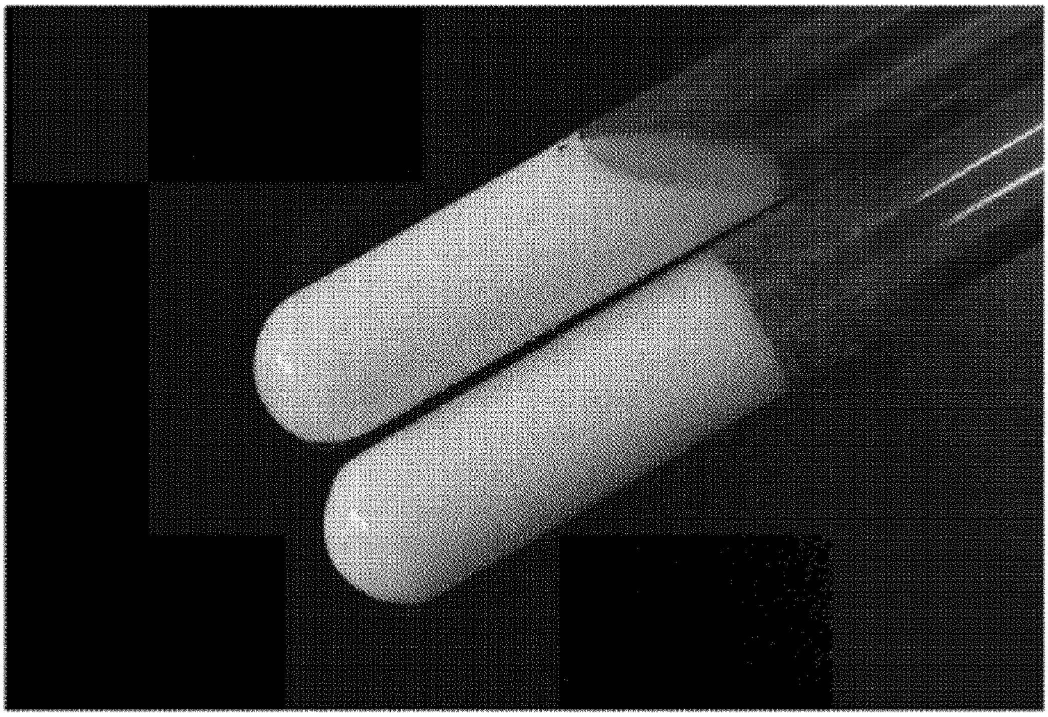

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1386539 A
- EP 1535999 A


### Non-patent literature cited in the description


- **YU P J ; CHOU C C.** Factors affecting the growth and production of milk clotting enzyme by Amylomyces rouxii in rice liquid medium[J. *Food Technology and Biotechnology,* 2005, vol. 43 (3), 283-288
- **HASHEM MA.** Purification and properties of a milk clotting enzyme produced by Penicillium oxalicum[J. *Bioresource Technology,* 2000, vol. 75 (3), 219-222
- **CAVALCANTI MTH ; MARTINEZ CR ; FURTADO VC ; NETO BB ; TEIXEIRA MF ; LIMA FILHO JL ; PORTO ALF.** Milk-clotting protease production by Nocardiopsis sp. in an inexpensive medium[J. *World Journal of Microbiology and Biotechnology,* 2005, vol. 21 (2), 151-154
- **HE X ; ZHANG W ; REN F ; GAN B ; GUO H.** Screening fermentation parameters of the milk-clotting enzyme produced by newly isolated Bacillus amyloliquefaciens D4 from the Tibetan Plateau in China[J. *Annals of Microbiology,* 2012, vol. 62 (1), 357-365
- **SHIEH C J ; PHAN THI LA ; SHIH I L.** Milk-clotting enzymes produced by culture of Bacillus subtilis natto [J. *Biochemical Engineering Journal,* 2009, vol. 43 (1), 85-91
- **DING Z Y ; LIU S P ; GU Z H ; ZHANG L ; ZHANG K C ; SHI G Y.** Production of milk-clotting enzyme by Bacillus subtilis B1 from wheat bran[J. *African Journal of Biotechnology,* 2011, vol. 10 (46), 9370-9378
- **LI Y ; LIANG S ; ZHI D ; CHEN P ; SU F ; LI H.** Purification and characterization of Bacillus subtilis milk-clotting enzyme from Tibet Plateau and its potential use in yak dairy industry [J. *European Food Research and Technology,* 2012, vol. 234 (4), 733-741
- **EL-BENDARY M A ; MOHARAM M E ; ALI T H.** Purification and Characterization of Milk Clotting Enzyme Produced by Bacillus sphaericus [J. *Journal of Applied Sciences Research,* 2007, vol. 3 (8), 695-699

<110> Bright Dairy & Food Co., Ltd

<120> Method for preparing fermentation broth extracts having chymosin activity and products thereof

<130> P4E1311033BC

<160> 3

<170> PatentIn version 3.5

<210> 1
<211> 1435
<212> DNA
<213> Paenibacillus sp.

<400> 1

```
tgcagtcgag cggagttgat agagtgcttg cactcttgag acttagcggc ggacgggtga      60
gtaacacgta ggcaacctgc cctcagact gggataacta ccggaaacgg tagctaatac      120
cggataatcg ttttcttctc ctgaagagac cgggaaagac ggagcaatct gtcactgagg     180
gatgggcctg cggcgcatta gctagttggt ggggtaacgg ctcaccaagg cgacgatgcg     240
tagccgacct gagagggtga tcggccacac tgggactgag acacggccca gactcctacg     300
ggaggcagca gtagggaatc ttccgcaatg gacgaaagtc tgacggagca acgccgcgtg     360
agtgatgaag gttttcggat cgtaaagctc tgttgccagg gaagaacgtc ttctagagta     420
actgctagaa gagtgacggt acctgagaag aaagccccgg ctaactacgt gccagcagcc     480
gcggtaatac gtagggggca agcgttgtcc ggaattattg ggcgtaaagc gcgcgcaggc     540
ggtcatttaa gtctggtgtt taatcccgaa gctcaacttc gggtcgcatc ggaaactgga     600
tgacttgagt gcagaagagg agagtggaat tccacgtgta gcggtgaaat gcgtagagat     660
gtggaggaac accagtggcg aaggcgactc tctgggctgt aactgacgct gaggcgcgaa     720
agcgtgggga gcaaacagga ttagataccc tggtagtcca cgccgtaaac gatgaatgct     780
aggtgttagg ggtttcgata cccttggtgc cgaagttaac acattaagca ttccgcctgg     840
ggagtacggt cgcaagactg aaactcaaag gaattgacgg ggacccgcac aagcagtgga     900
gtatgtggtt taattcgaag caacgcgaag aaccttacca ggtcttgaca tctgaatgac     960
cggtgcagag atgtaccttt tcttcggaac attcaagaca ggtggtgcat ggttgtcgtc    1020
agctcgtgtc gtgagatgtt gggttaagtc ccgcaacgag cgcaaccctt atgcttagtt    1080
gccagcacat catggtgggc actctaagca gactgccggt gacaaaccgg aggaaggtgg    1140
ggatgacgtc aaatcatcat gccccttatg acctgggcta cacacgtact acaatggtcg    1200
gtacaacggg aagcgaagcc gcgaggtgga gcgaatccta aaaagccgat ctcagttcgg    1260
attgcaggct gcaactcgcc tgcatgaagt cggaattgct agtaatcgcg gatcagcatg    1320

ccgcggtgaa tacgttcccg ggtcttgtac acaccgcccg tcacaccacg agagtttgca    1380
acacccgaag tcggtggggt aacccgcaag gagccagccg ccgaaggtgg                1430
```

<210> 2
<211> 16
<212> DNA
<213> Artificial Sequence

<220>
<223> Primer

<400> 2
taccttgtta cgactt 16

<210> 3
<211> 20
<212> DNA
<213> Artificial Sequence

<220>
<223> Primer

<400> 3
agagtttgat cctggctcag 20